# EUROPÄISCHE PATENTSCHRIFT

(11) **EP 3 074 974 B1**
(45) Veröffentlichungstag und Bekanntmachung des Hinweises auf die Patenterteilung: **06.02.2019**
(21) Anmeldenummer: 14796119.7
(22) Anmeldetag: 11.11.2014
(51) Int. Cl.: G10L 21/013, H04R 25/00, A61N 1/36

(54) **HÖRHILFEVORRICHTUNG MIT GRUNDFREQUENZMODIFIZIERUNG**
HEARING ASSISTANCE DEVICE WITH FUNDAMENTAL FREQUENCY MODIFICATION
DISPOSITIF DE CORRECTION AUDITIVE AVEC MODIFICATION DE LA FRÉQUENCE FONDAMENTALE

(30) Priorität: 28.11.2013 DE 102013224417
(43) Veröffentlichungstag der Anmeldung: 05.10.2016
(73) Patentinhaber: Fraunhofer-Gesellschaft zur Förderung der angewandten Forschung e.V., 80686 München (DE)
(72) Erfinder: HARCZOS, Tamas, 98704 Wolfsberg (DE)
(74) Vertreter: Zinkler, Franz
(86) Internationale Anmeldenummer: PCT/EP2014/074258
(87) Internationale Veröffentlichungsnummer: WO 2015/078689

(56) Entgegenhaltungen:
- EP-A1- 1 973 101
- CN-A- 102 779 526
- JP-A- 2013 117 556
- US-A1- 2005 107 843
- US-A1- 2010 125 222
- US-B1- 8 280 087

## Beschreibung

Ausführungsbeispiele der vorliegenden Erfindung beziehen sich auf eine Hörhilfevorrichtung. Weitere Ausführungsbeispiele der vorliegenden Erfindung beziehen sich auf ein Verfahren zur Verarbeitung eines Sprachsignals. Weitere Ausführungsbeispiele beziehen sich auf ein Computerprogramm zur Ausführung des Verfahrens zur Verarbeitung eines Sprachsignals auf einem Computer, mittels eines Prozessors, eines Mikrocontrollers oder einer vergleichbaren Vorrichtung. Weitere Ausführungsbeispiele beziehen sich auf eine Tonumfangserweiterung oder Tonhöhenbereichsverbreiterung für Hörhilfen und implantierbare Hörprothesen, um die Perzeption von prosodischen Merkmalen von Sprache zu verbessern.

Es wird geschätzt, dass bis jetzt (November 2013) weltweit ungefähr 250.000 Menschen Cochleaimplantate empfangen haben. Cochleaimplantate sind die häufigste Form von implantierbaren Hörprothesen. Cochleaimplantate ermöglichen es Menschen mit moderater bis schwerer sensorineuraler Schwerhörigkeit, Schall wahrzunehmen und ausreichende auditorische Information bereitzustellen, um ein einigermaßen ausreichendes Hörverständnis in ruhiger Umgebung zu ermöglichen. Während der Implantierung wird ein Elektroden-Array in die Cochlea (Innenohrschnecke) eingeführt, welches den Hörnerv mittels elektrischer Impulse stimuliert. Im Fall einer Schädigung des Hörnervs ist ein Hirnstammimplantat eine bessere Wahl als implantierbare Hörprothese, dessen Funktionsweise sehr ähnlich ist zu der eines Cochleaimplantats. Im Hinblick auf Sprachperzeption können Hirnstammimplantate allerdings typischerweise nicht die Effizienz von Cochleaimplantaten erreichen.

Im Gegensatz zu Elektroden-Arrays, die in implantierbaren Hörprothesen verwendet werden, wo jede Elektrode einem bestimmten Frequenzband entspricht, hat eine intakte Cochlea (Innenohrschnecke) mehr als 3000 innere Haarzellen, was eine viel bessere Frequenzauflösung ermöglicht (im Vergleich zu den ca. 10 - 50 Elektroden bzw. Frequenzbänder von üblichen Cochleaimplantaten).

Neben den Benutzern von implantierbaren Hörprothesen (implantierten Hörprothesen) verwenden weltweit ca. 40 Mio. Menschen mit geringem bis starkem Hörverlust nicht implantierte Hörgeräte. Während Hörgeräte in bestimmten Fällen (hauptsächlich in Verbindung mit geringem Hörverlust) das Hörvermögen auf einem hohen Standard wiederherstellen können, bleibt das geräteunterstützte Hören in den meisten Fällen immer noch hinter einem normalen Hörvermögen zurück.

Gegenwärtig verwenden Sprachprozessoren für Hörhilfen, Cochleaimplantate und Hirnstammimplantate eine Vielzahl von Vorverarbeitungsalgorithmen einschließlich automatischer Verstärkungs- und Empfindlichkeitssteuerung, Optimierung des Dynamikbereichs, Hintergrundgeräuschreduzierung, Windgeräuschreduzierung, etc.. Heutzutage liegt ein Fokus der Behandlung mittels Hörhilfen und implantierbaren Hörprothesen auf der Verbesserung der Perzeption von Wörtern und Sätzen. Allerdings ist die Erfassung von (begleitenden) Informationen wie der Intonation für Hörgerätbenutzer vermindert und oftmals nicht wahrnehmbar für Implantatbenutzer. Folglich können diese Benutzer wichtige Bestandteile der Hintergrundinformation, die in die Prosodie hineinkodiert sind, oder von dem Sprecher ausgedrückte Emotionen nicht wahrnehmen. Oft können Implantatbenutzer noch nicht einmal entscheiden, ob ein Satz eine Frage oder eine Aussage ist, was zu Unsicherheiten und sozialer Isolation führen kann

US-A-2005/107843 offenbart eine Hörhilfevorrichtung nach dem Oberbegriff des Anspruchs 1 und ein Verfahren nach dem Oberbegriff des Anspruchs 11.

EP-A-1 973 101 offenbart den durchschnittlichen Grundfrequenzwert als Alternative zu dem momentanen Grundfrequenzwert zu bestimmen.

Aufgabe der vorliegenden Erfindung ist es, die Wahrnehmung von sprachlicher Hintergrundinformation, wie z.B. Prosodie und/oder Emotionen, für die Benutzer von Hörhilfen zu verbessern und auf diese Weise die Teilnahme an Gesprächen mit Mitmenschen zu verbessern.

Diese Aufgabe und/oder weitere Aufgaben werden durch die unabhängigen Ansprüche gelöst.

Ausführungsbeispiele der vorliegenden Erfindung schaffen ein Hörhilfevorrichtung mit einer Frequenzanalyseeinrichtung, einer statistischen Auswertungseinrichtung, einem Grundfrequenzmodifizierers und einem Sprachsignalerzeuger. Die Frequenzanalyseeinrichtung ist konfiguriert, einen momentanen Grundfrequenzwert eines Sprachsignals für einen Zeitabschnitt des Sprachsignals zu bestimmen. Die statistische Auswertungseinrichtung ist konfiguriert, einen durchschnittlichen Grundfrequenzwert des Sprachsignals über mehrere Zeitabschnitte zu bestimmen. Der Grundfrequenzmodifizierer ist konfiguriert, den momentanen Grundfrequenzwert derart zu einem modifizierten Grundfrequenzwert zu modifizieren, dass eine Differenz oder ein Quotient des momentanen Grundfrequenzwerts zum durchschnittlichen Grundfrequenzwert gemäß einer bestimmten Funktion geändert wird, um so einen Frequenzbereich zu verändern, innerhalb dem der Grundfrequenzwert variiert. Der Sprachsignalerzeuger ist konfiguriert, ein hinsichtlich der Grundfrequenz modifiziertes Sprachsignal auf der Grundlage des modifizierten Grundfrequenzwerts zu erzeugen.

Ausführungsbeispiele der vorliegenden Erfindung basieren auf der Erkenntnis, dass aufgrund der relativ groben Frequenzauflösung von Hörhilfen und insbesondere implantierbaren Hörprothesen die Wahrnehmung von sprachlichen Nebeninformationen wie Prosodie (u.a. Sprachmelodie und dadurch übertragene Emotionen) für die Benutzter solcher Hörhilfen nur sehr eingeschränkt möglich ist. Insbesondere kann es sein, dass ein von einer bestimmten Person gesprochener Satz nur über eine geringe Anzahl von Elektroden wiedergegeben wird, da die Sprache und insbesondere die Grundfrequenz nur in einem begrenzten Frequenzbereich variiert, obwohl ein Normalhörender durchaus eine deutliche Änderung der Tonhöhe feststellen kann. Aufgrund der geringen Anzahl der verwendeten Elektroden hat dagegen vor allem ein Benutzer einer implantierten Hörprothese häufig Schwierigkeiten, Änderungen der Tonhöhe zu detektieren, welche unter anderem für eine Unterscheidung von Fragen und Aussagen, sowie für die Feststellung von Satzgrenzen wichtig sind. Die vorliegende Erfindung schlägt zur Abhilfe vor, den Frequenzbereich zu strecken, innerhalb dem die Grundfrequenz eines bestimmten Sprechers zum Beispiel zur Andeutung von Fragen, Aussagen und Satzgrenzen variiert, wobei jedoch auf eine aufwändige und häufig fehlerhafte syntaktische Analyse des Sprachsignals verzichtet werden kann. Somit basieren zumindest einige Ausführungsbeispiele der vorliegenden Erfindung auch auf der zusätzlichen oder alternativen Erkenntnis, dass im alltäglichen Gebrauch einer Hörhilfe die eigentliche Schwierigkeit wohlmöglich darin liegt, bei einem Sprachsignal mittels computerimplementierter Algorithmen eine zuverlässige syntaktische Analyse durchzuführen, um in der Folge eine Modifizierung der Grundfrequenz durchzuführen. Im Gegensatz zu diesen Anforderungen des alltäglichen Gebrauch einer Hörhilfe haben wissenschaftliche Studien auf diesem Gebiet stets eine manuelle Anpassung der Grundfrequenz von manuell ausgewählten Satzteilen vorgenommen, um zum einen wohldefinierte Testdaten zur Verfügung zu haben und zum anderen um den Effekt einer Grundfrequenzvariation auf das Verständnisvermögen von Hörhilfebenutzern zu untersuchen. Im Gegensatz dazu schlägt die vorliegende Erfindung nun vor, die Tonhöhenvariationen der Grundfrequenz, die im ursprünglichen Sprachsignal vorhanden sind, zu vergrößern (zu verstärken), so dass die Schwankungsbreite der Grundfrequenz eines bestimmten Sprechers vergrößert wird. Die vorliegende Erfindung löst somit unter anderem das Problem, wie die Hörhilfe feststellen kann, welche Zeitabschnitte des Sprachsignals auf welche Weise hinsichtlich ihrer Grundfrequenz verändert werden sollen (Grundfrequenz heraufsetzen, Grundfrequenz herabsetzen, Grundfrequenz im Wesentlichen beibehalten). Die vorliegende Erfindung leistet dies, indem sie für den aktuellen Sprecher und dessen aktuelle Sprechweise (z.B. neutral, ruhig, aufgeregt, freudig erregt, empört, etc.) eine durchschnittliche Grundfrequenz ermittelt. Diese durchschnittliche Grundfrequenz dient in der Folge wie eine Bezugsfrequenz (wie ein "Dreh- und Angelpunkt") für eine Modifikation der Grundfrequenz.

Die Modifikation des Grundfrequenzwerts dient dazu, einen modifizierten Grundfrequenzwert zu ermitteln. Die Modifikation kann mittels einer Funktion oder (mathematischen) Abbildung erfolgen, in die eine Differenz zwischen des momentanen Grundfrequenzwerts und des durchschnittlichen Grundfrequenzwerts als Argument eingeht. Die Funktion oder (mathematische) Abbildung kann parametrisierbar sein mittels eines Parameters oder mehrerer Parameter. Als Beispiel für einen Parameter sei ein Tonhöhenbereichsfaktor (engl. "pitch range factor" PRF) genannt, der angibt, um wie viel die Differenz zwischen momentanem Grundfrequenzwert und durchschnittlichem Grundfrequenz skaliert oder gestreckt werden soll. Anstelle der Differenz ist auch eine andere Relation zwischen dem momentanen Grundfrequenzwert und dem durchschnittlichen Grundfrequenzwert möglich, z. B. der Quotient.

Entsprechend einigen Ausführungsbeispielen kann die Hörhilfevorrichtung weiter eine Einrichtung zur Klassifizierung von stimmhaften Zeitabschnitten und stimmlosen Zeitabschnitten umfassen, wobei der Frequenzanalyseeinreichtung und die statistische Auswertungseinrichtung konfiguriert sind, die Bestimmung des momentanen Grundfrequenzwert und des durchschnittlichen Grundfrequenzwerts anhand von als stimmhaft klassifizierten Zeitabschnitten des Sprachsignals durchzuführen. In vielen Fällen macht eine Ermittlung und Änderung der Grundfrequenz nur innerhalb von stimmhaften Zeitabschnitten des Sprachsignals Sinn, sodass durch eine Unterscheidung zwischen stimmhaften und stimmlosen Zeitabschnitten eine Verfälschung der Ermittlung des momentanen Grundfrequenzwerts und des durchschnittlichen Grundfrequenzwerts durch stimmhafte Anteile weitgehend vermieden werden kann.

Entsprechend einigen Ausführungsbeispielen kann die Frequenzanalyseeinrichtung Teil einer Einrichtung zur linearen Prädiktionscodierungsanalyse (LPC) sein und der Sprachsignalerzeuger eine Einrichtung zur linear Prädiktionscodierungssynthese sein. Die lineare Prädiktionscodierung modelliert relativ realistisch die natürliche Erzeugung eines Sprachsignals durch einen Menschen. Der Grundfrequenzwert wird im Rahmen der linearen Prädiktionscodierungsanalyse als ein Signalparameter von typischerweise mehreren Signalparametern ermittelt. Bei der linearen Prädiktionscodierungssynthese wird aus den Signalparametern wieder ein Sprachsignal reproduziert, das im Wesentlichen mit dem ursprünglichen Sprachsignal übereinstimmt oder sich zumindest nicht allzu stark von dem ursprünglichen Sprachsignal unterscheidet. Dabei wird die Grundfrequenz verwendet, um zunächst ein sogenanntes Quellensignal (engl. "source signal") zu erzeugen. In manchen Fällen wird für die Erzeugung des Quellensignals auch ein Restsignal (engl. "residual signal") hinzugezogen. Das Quellensignal wird dann gefiltert, wobei das entsprechende Filter (entsprechend dem Vokaltrakt des Sprechers, also dessen Rachen und Mundraum) mit Hilfe von Formant-Parametern modelliert wird. Am Filterausgang wird das auf diese Weise reproduzierte Sprachsignal (LPC-synthetisiertes Sprachsignal) ausgegeben. Da der Grundfrequenzwert im Rahmen der LPC-Analyse unabhängig von den Formant-Parametern in die Synthese eingeht, kann durch Modifizierung des Grundfrequenzwerts eine Tonhöhenänderung des reproduzierten Sprachsignals erreicht werden, ohne dass die Formantfrequenzen und/oder -amplituden des reproduzierten Sprachsignals wesentlich geändert werden. Da die Formantfrequenzen nicht verändert werden, bleiben insbesondere Vokale (a, e, i, o, u) und ähnliche Laute wie gewohnt verständlich. Weiterhin klingt die Stimme weiterhin natürlich, nur die Sprachmelodie erscheint ausgeprägter.

Entsprechend einigen Ausführungsbeispielen kann der Sprachsignalerzeuger auf einer Fast Fourier Transformation (FFT) oder PSOLA (engl. "pitch synchronous overlap and add") basieren. Auch diese Verfahren bieten im Prinzip die Möglichkeit, die Grundfrequenz unabhängig von den Formantfrequenzen in die Synthese des Sprachsignals einfließen zu lassen, so dass sich die Grundfrequenz des Sprachsignals zeitabschnittsweise variieren lässt, ohne die Formanten wesentlich zu verfälschen.

Entsprechend einigen Ausführungsbeispielen kann der Sprachsignalerzeuger konfiguriert sein, Formantfrequenzen des Sprachsignals im Wesentlichen nicht zu ändern. Wie bereits erwähnt, wird dadurch die Verständlichkeit vor allem von Vokalen und anderen stimmhaften oder teilweise stimmhaften Lauten beibehalten.

Entsprechend einigen Ausführungsbeispielen kann der Grundfrequenzmodifizierer konfiguriert sein, einen durchschnittlichen modifizierten Grundfrequenzwert im Wesentlichen identisch zu dem durchschnittlichen Grundfrequenzwert zu halten. Auf diese Weise hat der Hörhilfebenutzer weiterhin einen Anhaltspunkt zur Unterscheidung von verschiedenen Sprechern und deren Eigenschaften (Mann, Frau, Kind). Mit anderen Worten bleibt die Möglichkeit der Geschlechterunterscheidung und Sprecheridentifikation für den Hörhilfebenutzer im Wesentlichen erhalten, was für den Hörhilfebenutzer in Situationen mit zwei oder mehr Gesprächspartnern hilfreich sein kann.

Entsprechend einigen Ausführungsbeispielen kann die bestimmte Funktion, mittels welcher die Differenz oder der Quotient geändert wird, eine lineare Funktion mit einem Proportionalitätsfaktor (PRF) sein, so dass die Differenz oder der Quotient anhand des Proportionalitätsfaktor skaliert wird. Alternativ sind aber auch andere Funktionen möglich, zum Beispiel eine Sigmoidfunktion.

Entsprechend einigen Ausführungsbeispielen kann der Grundfrequenzmodifizierer konfiguriert sein, den modifizierten Grundfrequenzwert an zumindest einem von einem unteren Grenzwert oder einem oberen Grenzwert zu beschränken. In Situationen, in denen der Sprecher bereits eine ausgeprägte Sprachmelodie aufweist, ist es möglich, dass der Hörhilfebenutzer auch ohne eine Modifikation der Grundfrequenz genügend Information zu Prosodie und Emotionen in dem originalen Sprachsignal wahrnehmen kann, und/oder dass eine unbeschränkte Modifikation der Grundfrequenz zu übertriebenen Variationen der Tonhöhe in dem modifizierten Sprachsignal führen würde. Weiterhin kann auf diese Weise eine Begrenzung auf den hörbaren bzw. technisch realisierbaren Frequenzbereich erreicht werden, so dass die modifizierte Grundfrequenz zum Beispiel nicht unter 50 Hz oder sogar unter 0 Hz liegen kann.

Entsprechend einigen Ausführungsbeispielen kann die statistische Auswertungseinrichtung konfiguriert sein, eine zeitliche Konstanz der Grundfrequenzwerte von mehreren Zeitabschnitten zu ermitteln und den durchschnittlichen Grundfrequenzwert erst dann an den Grundfrequenzmodifizierer zu übermitteln, wenn die zeitliche Konstanz über einem Mindestwert liegt. Die zeitliche Konstanz kann zum Beispiel durch eine Standardabweichung der Grundfrequenzwerte in einer Mindestanzahl von berücksichtigten Zeitabschnitten ausgedrückt werden (je größer die Standardabweichung, desto kleiner die zeitliche Konstanz, und umgekehrt).

Entsprechend einigen Ausführungsbeispielen kann die Hörhilfevorrichtung weiter einen Sprecherwechseldetektor umfassen, der konfiguriert ist zu detektieren, wenn innerhalb des Sprachsignals ein Wechsel von einem ersten Sprecher zu einem weiteren Sprecher stattgefunden hat, wobei die statische Auswertungseinrichtung und der Grundfrequenzmodifizierer konfiguriert sind, eine Datenverarbeitung für den ersten Sprecher solange auszusetzten, bis der Sprecherwechseldetektor einen Wechsel zurück zu dem ersten Sprecher detektiert. Bei einem davon abgeleiteten Ausführungsbeispiel kann ein Datenspeicher für mehrere Sprecher vorgesehen sein. Der Sprecherwechseldetektor kann konfiguriert sein, einen in dem Datenspeicher gespeicherten Sprecher anhand charakteristischer Eigenschaften (z.B. Grundfrequenz, Formantfrequenzen, Sprechgeschwindigkeit (z.B. durch den mittleren zeitlichen Abstand zwischen zwei stimmhaften Zeitabschnitten)) zu identifizieren und unmittelbar anschließend an die Identifikation die Datenverarbeitung innerhalb der statistischen Auswertungseinrichtung und des Grundfrequenzmodifiziers mit den im Datenspeicher gespeicherten Werte wieder aufzunehmen - im Wesentlichen verzögerungsfrei.

Ausführungsbeispiele schaffen ein Verfahren zur Verarbeitung eines Sprachsignals. Das Verfahren umfasst das Bestimmen eines momentanen Grundfrequenzwerts eines Sprachsignals für einen Zeitabschnitt des Sprachsignals und das Bestimmen eines durchschnittlichen Grundfrequenzwerts des Sprachsignals über mehrere Zeitabschnitte. Der momentane Grundfrequenzwert wird derart zu einem modifizierten Grundfrequenzwert modifiziert, dass eine Differenz oder ein Quotient des momentanen Grundfrequenzwerts zum durchschnittlichen Grundfrequenzwert gemäß einer bestimmten Funktion geändert wird, um so einen Frequenzbereich zu verändern, innerhalb dem der Grundfrequenzwert variiert. Das Verfahren umfasst weiterhin das Erzeugen eines hinsichtlich der Grundfrequenz modifizierten Sprachsignals auf der Grundlage des modifizierten Grundfrequenzwerts.

Ausführungsbeispiele schaffen ein Computerprogramm zur Ausführung des Verfahrens zur Verarbeitung eines Sprachsignals mittels eines Computers, Prozessors, Mikrocontrollers oder einer anderen programmierbaren Signalverarbeitungsvorrichtung.

Ausführungsbeispiele der vorliegenden Erfindung werden nachfolgend anhand der beiliegenden Zeichnungen erläutert. Es zeigen:
Fig. 1 zeigt ein schematisches Blockschaltbild einer allgemeinen Übersicht über den vorgeschlagenen Tonhöhenbereichserweiterer;
Fig. 2 zeigt ein schematisches Blockschaltbild eines Ausführungsbeispiels des hierin vorgeschlagenen Tonhöhenbereichserweiterer, der LPC (lineare Prädiktionscodierung) verwendet;
Fig. 3 einen zeitlichen Verlauf eines Sprachsignals und mehrere Spektrogramme des Sprachsignals sowie des modifizierten Sprachsignals für unterschiedliche Werte des Tonhöhenbereichsfaktors PRF;
Fig. 4 zeigt einen schematischen Verlauf einer Grundfrequenz und einer modifizierten Grundfrequenz für eine lineare Tonhöhenbereichsskalierung mit dem Tonhöhenbereichsfaktors PRF=40% (tatsächlich eine Verkleinerung des Tonhöhenbereichs);
Fig. 5 zeigt einen schematischen Verlauf einer Grundfrequenz und einer modifizierten Grundfrequenz für eine lineare Tonhöhenbereichsskalierung mit dem Tonhöhenbereichsfaktors PRF=150%;
Fig. 6 zeigt einen schematischen Verlauf einer Grundfrequenz und einer modifizierten Grundfrequenz für eine lineare Tonhöhenbereichsskalierung mit dem Tonhöhenbereichsfaktors PRF=200%; und
Fig. 7 zeigt ein schematisches Flussdiagramm eines Verfahrens zur Verarbeitung eines Sprachsignals gemäß einem Ausführungsbeispiel.

Bevor nachfolgend Ausführungsbeispiele der vorliegenden Erfindung anhand der beiliegenden Figuren im Detail erläutert werden, wird darauf hingewiesen, dass gleiche oder gleichwirkende Elemente oder Strukturen mit gleichen Bezugszeichen versehen sind, sodass die Beschreibung derer aufeinander anwendbar bzw. austauschbar ist.

Im Rahmen dieser Beschreibung und der Ansprüche bezeichnet der Begriff "Hörhilfe" den Oberbegriff für technische Vorrichtungen zur Verbesserung des Hörvermögens von schwerhörigen und/oder hörgeschädigten Menschen. Innerhalb der Hörhilfen lassen sich als Untergruppen unter anderem die implantierbaren Hörprothesen nennen, sowie die nicht implantierbaren Hörgeräte. Wie oben erwähnt, kann man innerhalb der Untergruppe der implantierbaren Hörprothesen unter anderem die Cochleaimplantate und die Hirnstammimplantate unterscheiden.

Gegenwärtig verwenden Sprachprozessoren für Hörhilfen (vor allem für Cochleaimplantate und Hirnstammimplantate) eine Vielzahl von Vorverarbeitungsalgorithmen einschließlich automatischer Verstärkungs- und Empfindlichkeitssteuerung, Optimierung des Dynamikbereichs, Hintergrundgeräuschreduzierung, Windgeräuschreduzierung, etc.. Jedoch ist zurzeit kein Vorverarbeitungsalgorithmus zur Verbesserung von prosodischen Hinweisen bekannt und folglich zur Verbesserung der Perzeption von Prosodie. Das Verfahren und die Vorrichtung, die hierin beschrieben werden, füllen diese Lücke.

In der Linguistik umfasst Prosodie die Aspekte Rhythmus, Betonung und Intonation von Sprache. Während der Rhythmus und die Betonung einigermaßen gut von geräteunterstützten hörgeschädigten Zuhörern wahrgenommen werden, beginnt die auditorische und audiologische Forschung, einen speziellen Schwerpunkt darauf zu legen, die Gründe von schlechter Intonationswahrnehmung und deren negativen Implikationen zu klären.

Akustische Merkmale von Intonation sind hauptsächlich in Tonhöhenvariationen kodiert. Ausführungsformen der vorliegenden Erfindung stellen ein Verfahren und eine Vorrichtung bereit, um:
- akustische Merkmale von Intonation durch eine Verstärkung von Tonhöhenvariationen (engl. "pitch variations") zu verbessern,
- auf eine Weise, die in die Vorverarbeitungskette von Hörhilfen einzuschließen ist,
- was bedeutet, dass eine Block-für-Block Verarbeitung von digitalisierter Sprache möglich ist,
- und eine Geschlechts- und Sprechidentifikation für den Zuhörer ermöglicht, da die Stimme des Sprechers nicht verzerrt oder verfälscht wird;
- dies wird erreicht durch Ändern der Grundfrequenz (engl. "fundamental frequency") F0 und deren Harmonischen auf kongruente Weise
- und dadurch, dass Formantfrequenzen nicht signifikant geändert werden.

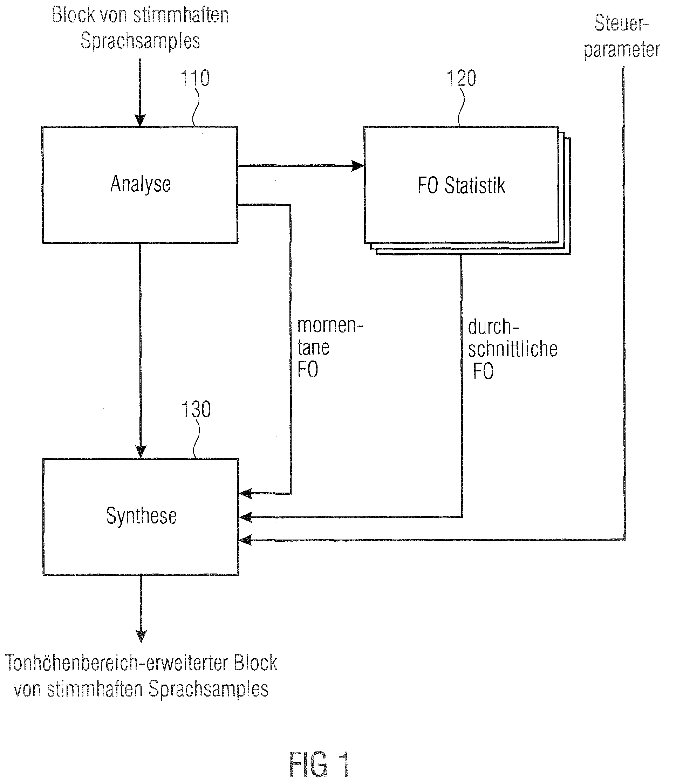

Fig. 1 zeigt eine allgemeine Übersicht über den vorgeschlagenen Tonhöhenbereichserweiterer. Als Eingangsdaten dient ein Block von stimmhaften Sprachsamples (engl. "block of voiced speech samples"). Dieser Block stellt einen Zeitabschnitt des Sprachsignals dar, welches zum Beispiel von einem Sprecher erzeugt wird und von einem Mikrophon der Hörhilfevorrichtung erfasst wird. Eine zeitliche Abtastung des Sprachsignals und eine anschließende Analog-Digital-Wandlung können durchgeführt werden, um eine Vielzahl von einzelnen digitalisierten Sprachsamples zu erhalten.

Die Tonhöhenbereichserweiterer, der Teil einer Hörhilfevorrichtung sein kann, umfasst eine Analyseeinrichtung 110, die insbesondere eine Frequenzanalyseeinrichtung sein kann. Diese Frequenzanalyseeinreichtung 110 ist konfiguriert, einen momentanen Grundfrequenzwert (momentane F0) eines Sprachsignals für einen Zeitabschnitt des Sprachsignals zu bestimmen, wobei der Zeitabschnitt hier dem Block von stimmhaften Sprachsamples entspricht. Es sind jedoch auch andere Beziehungen zwischen einem Block von Sprachsamples und einem Zeitabschnitt möglich. Die momentane Grundfrequenz F0 wird einer statistischen Auswertungseinrichtung 120 zugeführt, die konfiguriert ist, einen durchschnittlichen Grundfrequenzwert F0* des Sprachsignals über mehrere Zeitabschnitte zu bestimmen. Der momentane Grundfrequenzwert F0 und der durchschnittliche Grundfrequenzwert F0* werden an eine Syntheseeinrichtung oder einen Sprachsignalerzeuger 130 übergeben, welche als weitere Eingangsdaten zusätzliche Signalparameter bezüglich des Sprachsignals von der Frequenzanalyseeinrichtung 110 erhält. Auf der Grundlage des momentanen Grundfrequenzwerts F0, des durchschnittlichen Grundfrequenzwerts F0* und eines Steuerparameters wird nun eine modifizierte Grundfrequenz ermittelt. Eine Differenz oder ein Quotient des momentanen Grundfrequenzwerts F0 zum durchschnittlichen Grundfrequenzwert F0* wird dazu gemäß einer bestimmten, vordefinierten Funktion geändert, um so einen Frequenzbereich zu verändern, innerhalb dem der Grundfrequenzwert über eine Vielzahl von Zeitabschnitten variiert. Der Steuerparameter definiert dabei eine Stärke der Modifikation. Die Syntheseeinrichtung 130 verwendet den modifizierten Grundfrequenzwert für die Erzeugung eines modifizierten Sprachsignals innerhalb des entsprechenden Zeitabschnitts. Die Syntheseeinrichtung 130 gibt somit jeweils blockweise einen Tonhöhenbereich-erweiterten Block von stimmhaften Sprachsamples aus. Mit anderen Worten ist die Syntheseeinrichtung 130 konfiguriert, ein hinsichtlich der Grundfrequenz modifiziertes Sprachsignal auf der Grundlage des modifizierten Grundfrequenzwerts zu erzeugen.

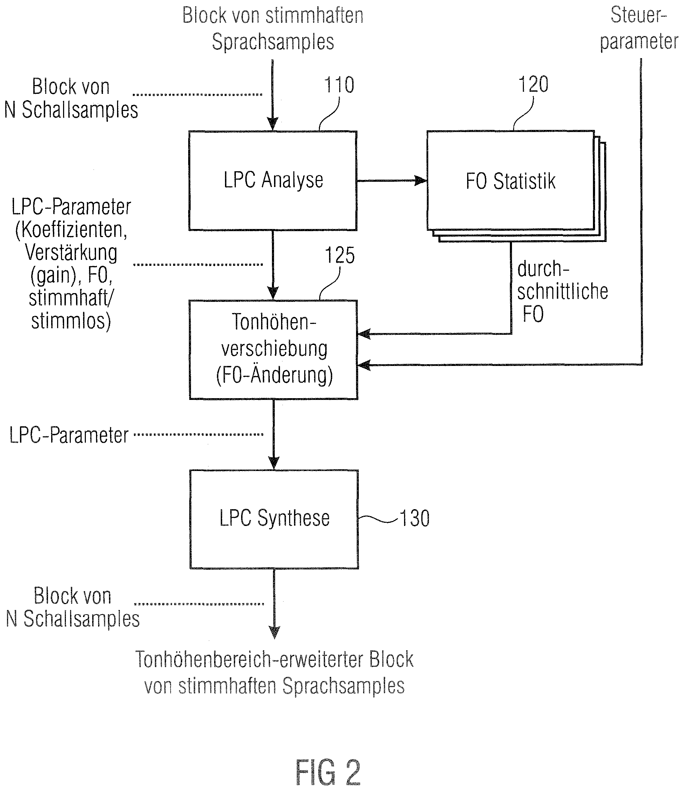

Fig. 2 zeigt ein schematisches Blockschaltbild eines Ausführungsbeispiels des vorgeschlagenen Verfahrens, welches lineare Prädiktionscodierung (LPC) verwendet. Wie in Fig. 1 wird ein Block von stimmhaften Sprachsamples von der Frequenzanalyseeinrichtung 110 analysiert, um einen momentanen Grundfrequenzwert zu bestimmen. Der Block kann zum Beispiel die Größe N haben, also N Schallsamples umfassen. Die Frequenzanalyseeinrichtung 110 ist in Fig. 2 eine LPC-Analyse, welche neben der für den aktuellen Block gültigen momentanen Grundfrequenz des Sprachsignals auch noch eine Reihe weiterer LPC Parameter liefert, insbesondere: Koeffizienten, Verstärkung (eng. "gain"), Information hinsichtlich der Stimmhaftigkeit des entsprechenden Zeitabschnitts oder Blocks des Sprachsignals. Die LPC-Parameter werden von der Analyseeinrichtung 110 an einen Grundfrequenzmodifizierer 125 übergeben, der eine Tonhöhenverschiebung bzw. Grundfrequenzänderung (F0-Änderung) vornimmt. Zu diesem Zweck erhält der Grundfrequenzmodifizierer 125 auch den durchschnittlichen Grundfrequenzwert F0* von der statistischen Auswertungseinrichtung 120. Wie in Fig. 1 wird auch zumindest ein Steuerparameter bereitgestellt, der das Ausmaß der Tonhöhenbereichsänderung festlegt. Die LPC-Parameter einschließlich des modifizierten Grundfrequenzwerts an den Sprachsignalerzeuger 130 übergeben, der in dem Ausführungsbeispiel von Fig. 2 eine LPC-Synthese durchführt. Aus den LPC-Parametern für den aktuellen Block erzeugt der Spracherzeuger einen Block von N Schallsamples des modifizierten Sprachsignals, bzw. den tonhöhenbereicherweiterten Block von stimmhaften Sprachsamples (engl. "pitch range extended block of voiced speech samples").

Bei dem in Fig. 2 gezeigten Ausführungsbeispiel wird somit ein Block digitalisierter Schallsamples durch die lineare Prädiktionscodierungstechnik analysiert. Ein Satz von Koeffizienten, Verstärkungsfaktor ("gain"), Grundfrequenz F0 und stimmhaft/stimmlos-Parametern wird für den kurzen Schallabschnitt bestimmt. Ausgehend von den momentanen Grundfrequenzen aufeinanderfolgender Blöcke (von stimmhaften Signalteilen) wird eine F0-Statistik aufgebaut. Insbesondere wird ein durchschnittlicher Grundfrequenzwert (welcher zum Beispiel der Median sein kann) des hereinkommenden Schalls berechnet, welcher mit F0* bezeichnet wird. Der Grundfrequenzmodifizierer bzw. die Tonhöhenverschiebungseinheit 125 unterhält auch eine durchschnittliche F0 (z.B. als F0** bezeichnet) der bereits verarbeiteten Daten. Der Tonhöhenbereich wird erweitert auf eine Weise, dass der Durchschnitt der Grundfrequenz ungefähr gleich bleibt, d.h. F0* ≈ F0**, aber die Differenz zwischen F0* und der momentaten Grundfrequenz F0 gemäß dem Faktor PRF skaliert wird. PRF=100% bedeutet demnach, dass keine Änderung erfolgt, wohingegen PRF=200% bedeutet, dass der F0-Bereich (maximale F0 - minimale F0) sich verdoppelt. Für hohe PRF-Werte kann es nötig sein, die minimal-mögliche und/oder maximalmögliche F0 zu begrenzen oder limitieren. Weiterhin kann es nötig sein, zunächst eine F0-Statistik aufzubauen (über bis zu mehrere hundert Millisekunden), ohne tatsächlich die Tonhöhe zu ändern. Das System kann gegebenenfalls dahingehend erweitert werden, dass es die Verarbeitung (insbesondere die Änderung der Grundfrequenz und das Fortschreiben der Statistik) umgeht, wenn mehrere Sprecher gleichzeitig sprechen. Eine weitere mögliche Erweiterung des Systems kann darin bestehen, dass das System plötzliche Sprecherwechsel detektiert.

Anstelle von LPC (wie in Fig. 2 gezeigt) können auch verschiedene andere formantbewahrende Tonhöhenänderungsmethoden verwendet werden. Diese können auf FFT (engl. "Fast Fourier Transform"), PSOLA (engl. "pitch synchronous overlap and add") oder anderen Techniken basieren.

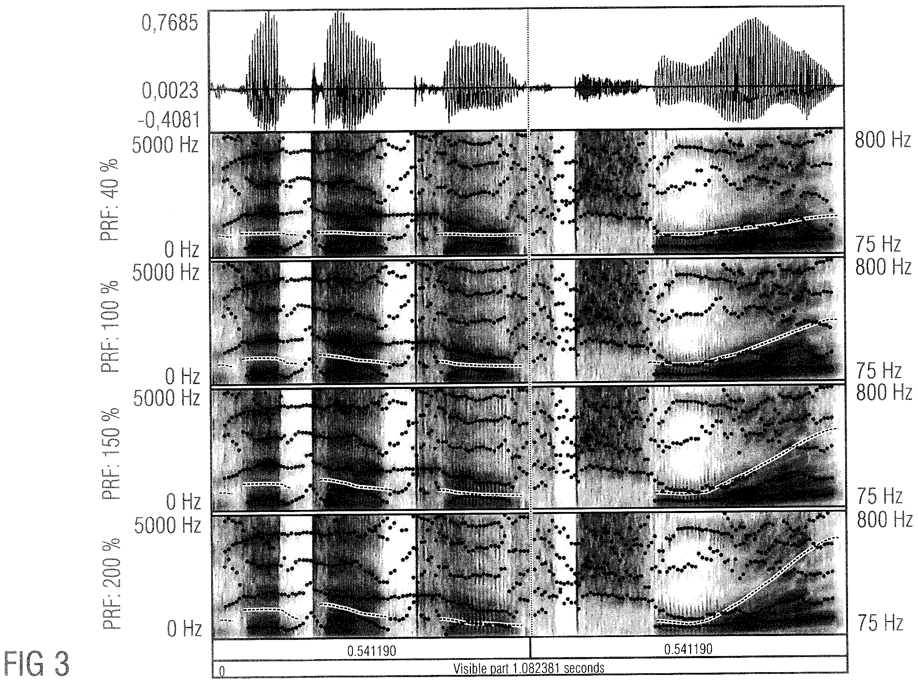

In den Ausführungsbeispielen gemäß den Fig. 1 und 2 spezifiziert der Steuerparameter das Ausmaß der Erweiterung des Tonhöhenbereichs, wie beispielhaft dargestellt in Fig. 3. Der Steuerparameter kann zum Beispiel ein Tonhöhenbereichsfaktor PRF (engl. "pitch range factor") sein.

Fig. 3 zeigt im oberen Teil den zeitlichen Signalverlauf eines Sprachsignals, das den kurzen deutschen Satz "Britta kauft Schuhe?" darstellt, der als Frage intoniert wurde (Grundfrequenz geht zur letzten Silbe hin nach oben). Die vier unteren Teildiagramme zeigen Spektrogramme der sprachlichen Äußerung nach Verarbeitung mit unterschiedlichen Werten für den Tonhöhenbereichsfaktor PRF als Steuerparameter, nämlich PRF=40% (Verkleinerung des Tonhöhenbereichs der Grundfrequenz), PRF=100% (keine Änderung des originalen Sprachsignals), PRF=150% (moderate Vergrößerung des Tonhöhenbereichs der Grundfrequenz) und PRF=200% (deutliche Vergrößerung des Tonhöhenbereichs der Grundfrequenz). Die Verläufe der Formantfrequenzen sind durch schwarze Punkte angedeutet. Für die Spektrogramme und die Formantfrequenzen gilt die linke Frequenzskala von 0 Hz bis 5000 Hz. Die Verläufe der Grundfrequenzen sind durch weiße Linien angedeutet (rechte Frequenzskala von 75 Hz bis 800Hz). Der Verlauf der Grundfrequenz ist nur für Zeitabschnitte gezeigt, die als stimmhaft klassifiziert wurden. In als stimmlos klassifizierten Zeitabschnitten ist kein Verlauf der Grundfrequenz gezeigt.

In Fig. 3 ist gut zu sehen, dass insbesondere in den Fällen PRF=150% und PRF=200% die Grundfrequenz zum Satzende hin deutlich stärker ansteigt, als im unveränderten Fall mit PRF=100%. Auf diese Weise kann auch eine Hörhilfeträger leichter erkennen, dass es sich bei dem vorliegenden Satz um eine Frage handelt. In Fig. 3 ist zudem auch zu erkennen, dass die Formantfrequenzen, die durch die schwarzen Punkte dargestellt werden, fast nicht geändert werden.

Ausgehend von einer früheren klinischen Studie und ersten Selbstversuchen des Erfinders kann eine signifikante Verbesserung der Wahrnehmung von Intonation erwartet werden bei Sprachsignalen, die gemäß dem vorgeschlagenen Verfahren verarbeitet werden. Die erwähnte klinische Studie verwendete manuell aufbereitete Sprachsignale, bei denen bestimmte Worte oder Silben bezüglich ihrer Tonhöhe manuell geändert wurden. Für denjenigen, der die manuelle Änderung durchführt, ist es bei Kenntnis der entsprechenden Sprache (Deutsch, Englisch, Chinesisch, Japanisch, ...) und grundlegenden Kenntnissen in Audiosignalverarbeitung kein Problem, das entsprechende Wort in einem grafisch dargestellten Signalverlauf zu markieren und diesen Signalabschnitt einer linearen Prädiktionscodierung mit modifizierter Grundfrequenz zu unterziehen. Die entsprechende Person greift für die manuelle Aufbereitung insbesondere auf ihre Kenntnisse der Semantik und Syntax der entsprechenden Sprache zurück. Bei der Anwendung in einer Hörhilfe ist es dagegen wünschenswert, wenn auf den hohen Rechenaufwand und auf die hohen Anforderungen an gespeicherten Daten oder der Datenmenge verzichtet werden kann, die mit einer computergestützten Semantik- und Syntaxanalyse einhergehen würde. Zudem müsste bei einer computergestützten Semantik- und Syntaxanalyse für jede Sprache (Deutsch, Englisch, ...) ein anderes Programm ausgeführt werden oder zumindest eine andere Konfiguration geladen werden, was einerseits für den Hörhilfeträger lästig sein kann und andererseits einen hohen Aufwand bei der Programmierung und dem Anlernen der Semantik- und Syntaxanalyse bedeutet. Die vorgeschlagenen Erfindung ist dagegen universell und unabhängig von der Sprache des Sprechers einsetzbar, liefert zuverlässig das gewünschte Ergebnis und erfordert nur geringen zusätzlichen Signalverarbeitungsaufwand innerhalb der Hörhilfe.

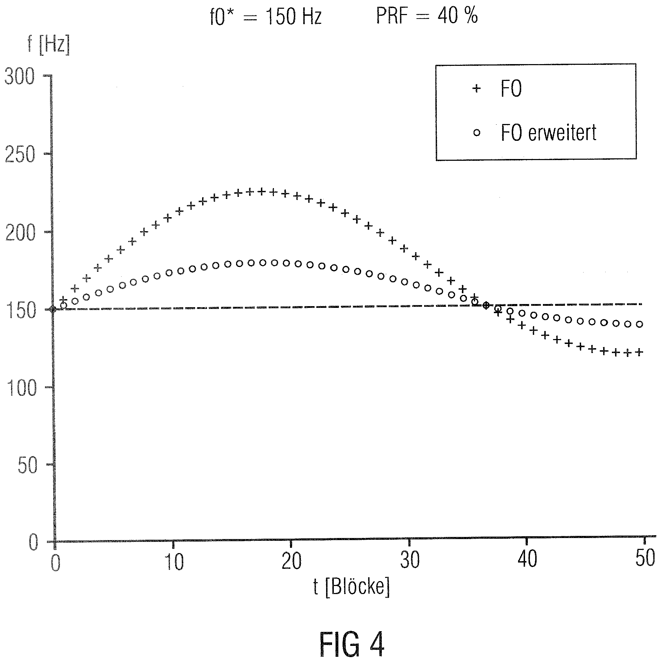

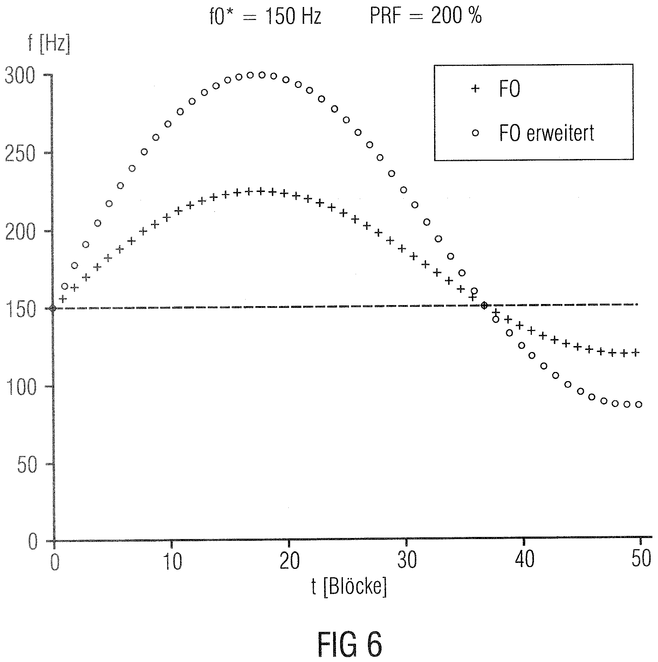

Die Figuren 4 bis 6 zeigen schematisch die Modifizierung der originalen Grundfrequenz F0 zur modifizierten Grundfrequenz F0* für die PRF-Werte 40%, 150% und 200%. Die Figuren zeigen jeweils 50 Blöcke auf der Zeitachse. Jeder Block kann N Samples umfassen. Die durchschnittliche Grundfrequenz liegt in allen Fällen, die in den Figuren 4 bis 6 dargestellt sind, konstant bei 150 Hz und ist als gestrichelte Linie dargestellt. Es sei angemerkt, dass die Bestimmung der durchschnittlichen Grundfrequenz zum Beispiel als gleitender Durchschnitt implementiert sein könnte, so dass die durchschnittliche Grundfrequenz sich von einem Block zum nächsten Block (geringfügig) ändern kann.

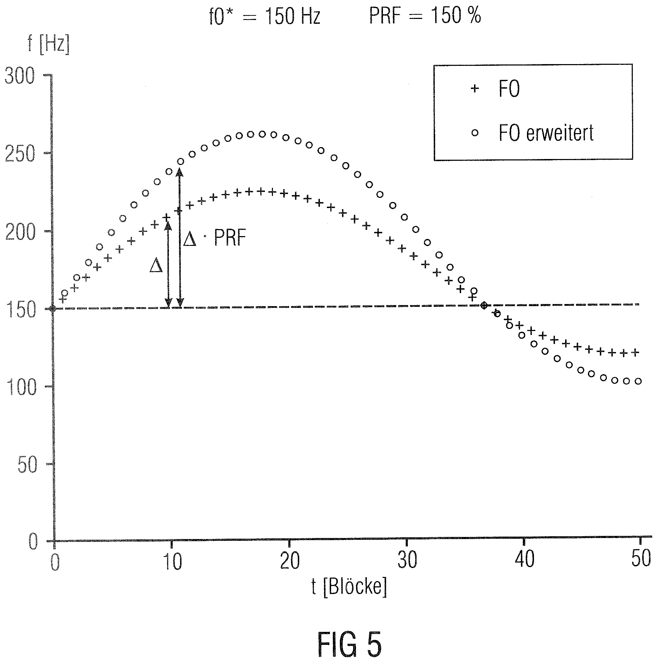

In Fig. 5 ist zur Veranschaulichung die Differenz Δ = F0 - F0* und die modifizierte Differenz Δ · PRF = (F0 - F0*). PRF grafisch dargestellt. Anstelle der Differenz kann auch eine andere Beziehung zwischen der momentanen Grundfrequenz F0 und der durchschnittlichen Grundfrequenz F0* verwendet werden, z.B. der Quotient F0/F0*.

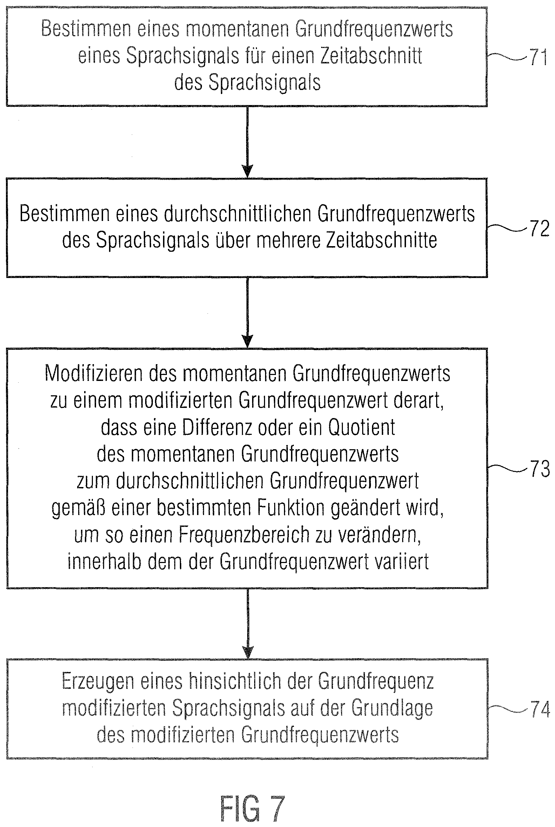

Fig. 7 zeigt ein schematisches Flussdiagramm eines Verfahrens zur Verarbeitung eines Sprachsignals gemäß Ausführungsbeispielen der vorliegenden Erfindung. In einem Schritt 71 wird ein momentaner Grundfrequenzwert F0 eines Sprachsignals für einen Zeitabschnitt des Sprachsignals bestimmt. In einem nachfolgenden Schritt 72 wird ein durchschnittlicher Grundfrequenzwert F0* des Sprachsignals über mehrere Zeitabschnitte bestimmt. Der momentane Grundfrequenzwert F0 wird dann in einem Schritt 73 zu einem modifizierten Grundfrequenzwert modifiziert. Zu diesem Zweck wird z.B. eine Differenz oder ein Quotient des momentanen Grundfrequenzwerts F0 zum durchschnittlichen Grundfrequenzwert F0* gemäß einer bestimmten Funktion geändert. Auf diese Weise kann ein Frequenzbereich (insbesondere die Breite des Frequenzbereichs) verändert werden, innerhalb dem der Grundfrequenzwert variiert. In einem Schritt 74 wird auf der Grundlage des modifizierten Grundfrequenzwerts ein hinsichtlich der Grundfrequenz modifiziertes Sprachsignal erzeugt.

Gemäß weiteren Ausführungsbeispielen kann das Verfahren eine Klassifizierung von stimmhaften Zeitabschnitten und stimmlosen Zeitabschnitten umfassen, wobei die Bestimmung des momentanen Grundfrequenzwerts und des durchschnittlichen Grundfrequenzwerts anhand von als stimmhaft klassifizierten Zeitabschnitten des Sprachsignals durchzuführen. Die als stimmlos klassifizierten Zeitabschnitte werden dagegen in der Regel nicht für die Bestimmung des momentanen Grundfrequenzwerts und des durchschnittlichen Grundfrequenzwerts herangezogen.

Die Bestimmung des momentanen Grundfrequenzwerts kann im Rahmen einer linearen Prädiktionscodierungsanalyse (LPC) erfolgen. Das Erzeugen des modifizierten Sprachsignals kann gemäß einer linearen Prädiktionscodierungssynthese erfolgen, wobei der modifizierte Grundfrequenzwert verwendet wird. Gemäß dazu alternativen Ausführungsformen kann die Erzeugung des modifizierten Sprachsignals auf einer Fast Fourier Transformation oder PSOLA (pitch synchronous overlap and add) basieren.

Gemäß Ausführungsbeispielen werden Formantfrequenzen des Sprachsignals im Wesentlichen nicht geändert. Dies betrifft typischerweise vor allem das Erzeugen des modifizierten Sprachsignals.

Gemäß Ausführungsbeispielen kann im Rahmen des Schritts des Modifizierens des momentanen Grundfrequenzwerts darauf geachtet werden, dass ein durchschnittlicher modifizierter Grundfrequenzwert im Wesentlichen identisch zu dem durchschnittlichen Grundfrequenzwert gehalten wird.

Die bestimmte Funktion, mittels welcher die Differenz oder der Quotient geändert wird, kann zum Beispiel eine lineare Funktion mit einem Proportionalitätsfaktor (PRF) sein, so dass die Differenz oder der Quotient anhand des Proportionalitätsfaktor skaliert wird.

Gemäß Ausführungsbeispielen kann der modifizierte Grundfrequenzwert an zumindest einem von einem unteren Grenzwert oder einem oberen Grenzwert beschränkt werden, um eine übermäßige oder übertriebene Änderung der Grundfrequenz zu verhindern.

Gemäß Ausführungsbeispielen kann das Verfahren weiterhin eine Ermittlung einer zeitlichen Konstanz der Grundfrequenzwerte innerhalb von mehreren Zeitabschnitten umfassen. Erst wenn die zeitliche Konstanz über einem voreingestellten (konfigurierten) Mindestwert liegt wird die Modifizierung des momentanen Grundfrequenzwerts auf der Grundlage des durchschnittlichen Grundfrequenzwerts vorgenommen. Auf diese Weise kann verhindert werden, dass Übergangseffekte zum Beispiel bei einem Sprecherwechsel die Modifizierung des Grundfrequenzwerts auf unerwünschte Weise beeinflussen.

Gemäß weiteren möglichen Ausführungsbeispielen kann das Verfahren einen Schritt zur Detektion eines Sprecherwechsels umfassen. Somit kann detektiert werden, wenn innerhalb des Sprachsignals ein Wechsel von einem ersten Sprecher zu einem weiteren Sprecher stattgefunden hat. Die Bestimmung der durchschnittlichen Grundfrequenz und das Modifizieren des momentanen Grundfrequenzwerts für den ersten Sprecher kann solange ausgesetzt werden, bis ein weiterer Sprecherwechsel zurück zu dem ersten Sprecher detektiert wurde.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar. Einige oder alle der Verfahrensschritte können durch einen Hardware-Apparat (oder unter Verwendung eines Hardware-Apparats), wie zum Beispiel einen Mikroprozessor, einen programmierbaren Computer oder eine elektronische Schaltung ausgeführt werden. Bei einigen Ausführungsbeispielen können einige oder mehrere der wichtigsten Verfahrensschritte durch einen solchen Apparat ausgeführt werden.

Manche Ausführungsbeispiele gemäß der Erfindung umfassen also einen Datenträger, der elektronisch lesbare Steuersignale aufweist, die in der Lage sind, mit einem programmierbaren Computersystem derart zusammenzuwirken, dass eines der hierin beschriebenen Verfahren durchgeführt wird.

Allgemein können Ausführungsbeispiele der vorliegenden Erfindung als Computerprogrammprodukt mit einem Programmcode implementiert sein, wobei der Programmcode dahin gehend wirksam ist, eines der Verfahren durchzuführen, wenn das Computerprogrammprodukt auf einem Computer abläuft.

Der Programmcode kann beispielsweise auch auf einem maschinenlesbaren Träger gespeichert sein.

Andere Ausführungsbeispiele umfassen das Computerprogramm zum Durchführen eines der hierin beschriebenen Verfahren, wobei das Computerprogramm auf einem maschinenlesbaren Träger gespeichert ist.

Mit anderen Worten ist ein Ausführungsbeispiel des erfindungsgemäßen Verfahrens somit ein Computerprogramm, das einen Programmcode zum Durchführen eines der hierin beschriebenen Verfahren aufweist, wenn das Computerprogramm auf einem Computer abläuft.

Ein weiteres Ausführungsbeispiel der erfindungsgemäßen Verfahren ist somit ein Datenträger (oder ein digitales Speichermedium oder ein computerlesbares Medium), auf dem das Computerprogramm zum Durchführen eines der hierin beschriebenen Verfahren aufgezeichnet ist.

Ein weiteres Ausführungsbeispiel des erfindungsgemäßen Verfahrens ist somit ein Datenstrom oder eine Sequenz von Signalen, der bzw. die das Computerprogramm zum Durchführen eines der hierin beschriebenen Verfahren darstellt bzw. darstellen. Der Datenstrom oder die Sequenz von Signalen kann bzw. können beispielsweise dahin gehend konfiguriert sein, über eine Datenkommunikationsverbindung, beispielsweise über das Internet, transferiert zu werden.

Ein weiteres Ausführungsbeispiel umfasst eine Verarbeitungseinrichtung, beispielsweise einen Computer oder ein programmierbares Logikbauelement, die dahin gehend konfiguriert oder angepasst ist, eines der hierin beschriebenen Verfahren durchzuführen.

Ein weiteres Ausführungsbeispiel umfasst einen Computer, auf dem das Computerprogramm zum Durchführen eines der hierin beschriebenen Verfahren installiert ist.

Ein weiteres Ausführungsbeispiel gemäß der Erfindung umfasst eine Vorrichtung oder ein System, die bzw. das ausgelegt ist, um ein Computerprogramm zur Durchführung zumindest eines der hierin beschriebenen Verfahren zu einem Empfänger zu übertragen. Die Übertragung kann beispielsweise elektronisch oder optisch erfolgen. Der Empfänger kann beispielsweise ein Computer, ein Mobilgerät, ein Speichergerät oder eine ähnliche Vorrichtung sein. Die Vorrichtung oder das System kann beispielsweise einen Datei-Server zur Übertragung des Computerprogramms zu dem Empfänger umfassen.

Bei manchen Ausführungsbeispielen kann ein programmierbares Logikbauelement (beispielsweise ein feldprogrammierbares Gatterarray, ein FPGA) dazu verwendet werden, manche oder alle Funktionalitäten der hierin beschriebenen Verfahren durchzuführen. Bei manchen Ausführungsbeispielen kann ein feldprogrammierbares Gatterarray mit einem Mikroprozessor zusammenwirken, um eines der hierin beschriebenen Verfahren durchzuführen. Allgemein werden die Verfahren bei einigen Ausführungsbeispielen seitens einer beliebigen Hardwarevorrichtung durchgeführt. Diese kann eine universell einsetzbare Hardware wie ein Computerprozessor (CPU) sein oder für das Verfahren spezifische Hardware, wie beispielsweise ein ASIC.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

## Patentansprüche

1. Hörhilfevorrichtung umfassend:
eine Frequenzanalyseeinrichtung, die konfiguriert ist, einen momentanen Grundfrequenzwert eines Sprachsignals für einen Zeitabschnitt des Sprachsignals zu bestimmen und
einen Sprachsignalerzeuger, der konfiguriert ist, ein hinsichtlich der Grundfrequenz modifiziertes Sprachsignal auf der Grundlage eines modifizierten Grundfrequenzwerts zu erzeugen;
**gekennzeichnet durch**
eine statistische Auswertungseinrichtung, die konfiguriert ist, einen durchschnittlichen Grundfrequenzwert des Sprachsignals über mehrere Zeitabschnitte zu bestimmen und
einen Grundfrequenzmodifizierer, der konfiguriert ist, den momentanen Grundfrequenzwert derart zu dem modifizierten Grundfrequenzwert zu modifizieren, dass eine Differenz oder ein Quotient des momentanen Grundfrequenzwerts zum durchschnittlichen Grundfrequenzwert gemäß einer bestimmten Funktion geändert wird, um so einen Frequenzbereich zu verändern, innerhalb dem der Grundfrequenzwert variiert.

2. Hörhilfevorrichtung gemäß Anspruch 1, weiter umfassend eine Einrichtung zur Klassifizierung von stimmhaften Zeitabschnitten und stimmlosen Zeitabschnitten, wobei die Frequenzanalyseeinrichtung und die statistische Auswertungseinrichtung konfiguriert sind, die Bestimmung des momentanen Grundfrequenzwerts und des durchschnittlichen Grundfrequenzwerts anhand von als stimmhaft klassifizierten Zeitabschnitten des Sprachsignals durchzuführen.

3. Hörhilfevorrichtung gemäß Anspruch 1 oder 2, wobei der Sprachsignalerzeuger konfiguriert ist, Formantfrequenzen des Sprachsignals im Wesentlichen nicht zu ändern.

4. Hörhilfevorrichtung gemäß Anspruch einem der Ansprüche 1 bis 3, wobei der Grundfrequenzmodifizierer konfiguriert ist, einen durchschnittlichen modifizierten Grundfrequenzwert im Wesentlichen identisch zu dem durchschnittlichen Grundfrequenzwert zu halten.

5. Hörhilfevorrichtung gemäß einem der Ansprüche 1 bis 4, wobei die Frequenzanalyseeinrichtung Teil einer Einrichtung zur linearen Prädiktionscodierungsanalyse (LPC) ist und der Sprachsignalerzeuger eine Einrichtung zur linear Prädiktionscodierungssynthese ist.

6. Hörhilfevorrichtung gemäß einem der Ansprüche 1 bis 4, wobei der Sprachsignalerzeuger auf einer Fast Fourier Transformation oder PSOLA (pitch synchronous overlap and add) basiert.

7. Hörhilfevorrichtung gemäß einem der Ansprüche 1 bis 6, wobei die bestimmte Funktion, mittels welcher die Differenz oder der Quotient geändert wird, eine lineare Funktion mit einem Proportionalitätsfaktor (PRF) ist, so dass die Differenz oder der Quotient anhand des Proportionalitätsfaktor skaliert wird.

8. Hörhilfevorrichtung gemäß einem der Ansprüche 1 bis 7, wobei der Grundfrequenzmodifizierer konfiguriert ist, den modifizierten Grundfrequenzwert an zumindest einem von einem unteren Grenzwert oder einem oberen Grenzwert zu beschränken.

9. Hörhilfevorrichtung gemäß einem der Ansprüche 1 bis 8, wobei die statistische Auswertungseinrichtung konfiguriert ist, eine zeitliche Konstanz der Grundfrequenzwerte von mehreren Zeitabschnitten zu ermitteln und den durchschnittlichen Grundfrequenzwert erst dann an den Grundfrequenzmodifizierer zu übermitteln, wenn die zeitliche Konstanz über einem Mindestwert liegt.

10. Hörhilfevorrichtung gemäß einem der Ansprüche 1 bis 9, weiter umfassend einen Sprecherwechseldetektor, der konfiguriert ist zu detektieren, wenn innerhalb des Sprachsignals ein Wechsel von einem ersten Sprecher zu einem weiteren Sprecher stattgefunden hat, wobei die statische Auswertungseinrichtung und der Grundfrequenzmodifizierer konfiguriert sind, eine Datenverarbeitung für den ersten Sprecher solange auszusetzten, bis der Sprecherwechseldetektor einen Wechsel zurück zu dem ersten Sprecher detektiert.

11. Verfahren zur Verarbeitung eines Sprachsignals,
Bestimmen eines momentanen Grundfrequenzwerts eines Sprachsignals für einen Zeitabschnitt des Sprachsignals und
Erzeugen eines hinsichtlich der Grundfrequenz modifizierten Sprachsignals auf der Grundlage eines modifizierten Grundfrequenzwerts;
**gekennzeichnet durch**
Bestimmen eines durchschnittlichen Grundfrequenzwerts des Sprachsignals über mehrere Zeitabschnitte und
Modifizieren des momentanen Grundfrequenzwerts zu dem modifizierten Grundfrequenzwert derart, dass eine Differenz oder ein Quotient des momentanen Grundfrequenzwerts zum durchschnittlichen Grundfrequenzwert gemäß einer bestimmten Funktion geändert wird, um so einen Frequenzbereich zu verändern, innerhalb dem der Grundfrequenzwert variiert.

12. Computerprogramm mit einem Programmcode, der bei der Ausführung des Programms durch einen Computer diesen veranlasst, das Verfahren nach Anspruch 11 auszuführen.

## Claims

1. Hearing aid apparatus, including:
a frequency analysis device that is configured to determine an instantaneous fundamental frequency value of a speech signal for a time portion of the speech signal; and
a speech signal generator that is configured to generate, on the basis of the modified fundamental frequency value, a speech signal modified with regard to the fundamental frequency;
**characterized by**
a statistical evaluation device that is configured to determine an average fundamental frequency value of the speech signal over several time portions; and
a fundamental frequency modifier that is configured to modify the instantaneous fundamental frequency value to the modified fundamental frequency value such that a difference or a quotient of the instantaneous fundamental frequency value is changed to the average fundamental frequency value according to a specific function, in order to thus modify a frequency range within which the fundamental frequency value varies.

2. Hearing aid apparatus according to claim 1, further including a device for classifying voiced time portions and unvoiced time portions, the frequency analysis device and the statistical evaluation device being configured to determine the instantaneous fundamental frequency value and the average fundamental frequency value by means of time portions of the speech signal that are classified as voiced.

3. Hearing aid apparatus according to claim 1 or 2, wherein the speech signal generator is configured to not substantially change formant frequencies of the speech signal.

4. Hearing aid apparatus according to any one of claims 1 to 3, wherein the fundamental frequency modifier is configured to keep an average modified fundamental frequency value substantially identical with the average fundamental frequency value.

5. Hearing aid apparatus according to any one of claims 1 to 4, wherein the frequency analysis device is part of a device for linear predictive coding analysis (LPC) and the speech signal generator is a device for linear predictive coding synthesis.

6. Hearing aid apparatus according to any one of claims 1 to 4, wherein the speech signal generator is based on a Fast Fourier Transformation or PSOLA (pitch synchronous overlap and add).

7. Hearing aid apparatus according to any one of claims 1 to 6, wherein the specific function by means of which the difference or the quotient is changed is a linear function having a proportionality factor (PRF), so that the difference or the quotient is scaled by means of the proportionality factor.

8. Hearing aid apparatus according to any one of claims 1 to 7, wherein the fundamental frequency modifier is configured to limit the modified fundamental frequency value at at least one of a lower limit value and an upper limit value.

9. Hearing aid apparatus according to any one of claims 1 to 8, wherein the statistical evaluation device is configured to determine a temporal constancy of the fundamental frequency values of several time portions and to transmit the average fundamental frequency value to the fundamental frequency modifier only when the temporal constancy is above a minimum value.

10. Hearing aid apparatus according to any one of claims 1 to 9, further including a speaker change detector that is configured to detect when a change from a first speaker to another speaker has occurred within the speech signal, the statistical evaluation device and the fundamental frequency modifier being configured to interrupt data processing for the first speaker until the speaker change detector detects a change back to the first speaker.

11. Method for processing a speech signal, comprising:
determining an instantaneous fundamental frequency value of a speech signal for a time portion of the speech signal and
generating, on the basis of the modified fundamental frequency value, a speech signal modified with regard to the fundamental frequency;
**characterized by**
determining an average fundamental frequency value of the speech signal over several time portions; and
modifying the instantaneous fundamental frequency value to the modified fundamental frequency value such that a difference or a quotient of the instantaneous fundamental frequency value is changed to the average fundamental frequency value according to a specific function in order to modify a frequency range within which the fundamental frequency value varies.

12. Computer program having a program code that, upon execution of the program by a computer, causes the same to perform the method according to claim 11.

## Revendications

1. Dispositif de correction auditive comprenant:
un moyen d'analyse de fréquence qui est configuré pour déterminer une valeur de fréquence de base momentanée d'un signal vocal pendant un segment de temps du signal vocal, et
un générateur de signal vocal qui est configuré pour générer, sur base d'une valeur de fréquence de base modifiée, un signal vocal modifié quant à la fréquence de base;
**caractérisé par**
un dispositif d'évaluation statistique qui est configuré pour déterminer une valeur de fréquence de base moyenne du signal vocal sur plusieurs segments de temps, et
un modificateur de fréquence de base qui est configuré pour modifier la valeur de fréquence de base momentanée à la valeur de fréquence de base modifiée de sorte telle qu'une différence ou un quotient de la valeur de fréquence de base momentanée par rapport à la valeur de fréquence de base moyenne soit modifié selon une certaine fonction de manière à modifier ainsi une plage de fréquences dans laquelle varie la valeur de fréquence de base.

2. Dispositif de correction auditive selon la revendication 1, comportant par ailleurs un moyen de classification de segments de temps vocalisés et de segments de temps non vocalisés, dans lequel le moyen d'analyse de fréquence et le moyen d'évaluation statistique sont configurés pour effectuer la détermination de la valeur de fréquence de base momentanée et de la valeur de fréquence de base moyenne à l'aide de segments de temps du signal vocal classifiés comme vocalisés.

3. Dispositif de correction auditive selon la revendication 1 ou 2, dans lequel le générateur de signal vocal est configuré pour substantiellement ne pas modifier les fréquences de formant du signal vocal.

4. Dispositif de correction auditive selon l'une des revendications 1 à 3, dans lequel le modificateur de fréquence de base est configuré pour maintenir une valeur de fréquence de base modifiée moyenne sensiblement identique à la valeur de fréquence de base moyenne.

5. Dispositif de correction auditive selon l'une des revendications 1 à 4, dans lequel le moyen d'analyse de fréquence fait partie d'un moyen d'analyse de codage à prédiction linéaire (LPC) et le générateur de signal vocal est un moyen de synthèse de codage à prédiction linéaire.

6. Dispositif de correction auditive selon l'une des revendications 1 à 4, dans lequel le générateur de signal vocal est basé sur une Transformation de Fourier Rapide ou PSOLA (pitch synchronous overlap and add).

7. Dispositif de correction auditive selon l'une des revendications 1 à 6, dans lequel la fonction déterminée au moyen de laquelle est modifié la différence ou le quotient est une fonction linéaire avec un facteur de proportionnalité (PRF), de sorte que la différence ou le quotient soit mis à échelle à l'aide du facteur de proportionnalité.

8. Dispositif de correction auditive selon l'une des revendications 1 à 7, dans lequel le modificateur de fréquence de base est configuré pour limiter la valeur de fréquence de base modifiée à au moins l'une parmi une valeur limite inférieure ou une valeur limite supérieure.

9. Dispositif de correction auditive selon l'une des revendications 1 à 8, dans lequel le dispositif d'évaluation statistique est configuré pour déterminer une constance dans le temps des valeurs de fréquence de base de plusieurs segments de temps et pour ne transmettre la valeur de fréquence de base moyenne au modificateur de fréquence de base que lorsque la constance dans le temps est supérieure à une valeur minimale.

10. Dispositif de correction auditive selon l'une des revendications 1 à 9, comportant par ailleurs un détecteur de changement de locuteur qui est configuré pour détecter le moment où a eu lieu dans le signal vocal un changement d'un premier locuteur à un autre locuteur, dans lequel le moyen d'évaluation statique et le modificateur de fréquence de base sont configurés pour suspendre un traitement de données pour le premier locuteur jusqu'à ce que le détecteur de changement de locuteur détecte un changement de retour au premier locuteur.

11. Procédé de traitement d'un signal de parole,
déterminer une valeur de fréquence de base momentanée d'un signal vocal pour un segment de temps du signal vocal, et
générer un signal vocal modifié quant à la fréquence de base sur base d'une valeur de fréquence de base modifiée;
**caractérisé par** le fait de
déterminer une valeur de fréquence de base moyenne du signal vocal sur plusieurs segments de temps; et
modifier la valeur de fréquence de base momentanée à la valeur de fréquence de base modifiée, de sorte qu'une différence ou un quotient de la valeur de fréquence de base momentanée soit modifiée à la valeur de fréquence de base moyenne selon une fonction déterminée, pour modifier ainsi une plage de fréquences dans laquelle varie la valeur de fréquence de base.

12. Programme d'ordinateur avec un code de programme qui, lorsque le programme est exécuté sur un ordinateur, fait que ce dernier réalise le procédé selon la revendication 11.
